(19) Europäisches Patentamt
European Patent Office
Office européen des brevets

(11) Veröffentlichungsnummer: **0 271 795**
**A2**

(12) # EUROPÄISCHE PATENTANMELDUNG

(21) Anmeldenummer: **87118047.7**

(22) Anmeldetag: **07.12.87**

(51) Int. Cl.4: **C07D 487/04** , A61K 31/55 , //(C07D487/04,243:00,237:00)

Patentanspruch für folgende Vertragsstaat: ES + GR.

(30) Priorität: **15.12.86 GB 8629875**

(43) Veröffentlichungstag der Anmeldung: **22.06.88 Patentblatt 88/25**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Lawton, Geoffrey**
**109 Whitehill Road**
**Hitchin Herts.(GB)**
Erfinder: **Redshaw, Sally**
**29 Hertford Road**
**Stevenage Herts.(GB)**

(74) Vertreter: **Kellenberger, Marcus, Dr. et al**
**Grenzacherstrasse 124 Postfach 3255**
**CH-4002 Basel(CH)**

(54) **Octahydro-10-oxo-6H-pyridazo [1,2-a] [1,2] diazepin-Derivate, Zwischenprodukte und Verfahren zu deren Herstellung sowie diese enthaltende Arzneimittel.**

(57) Verbindungen der Formel

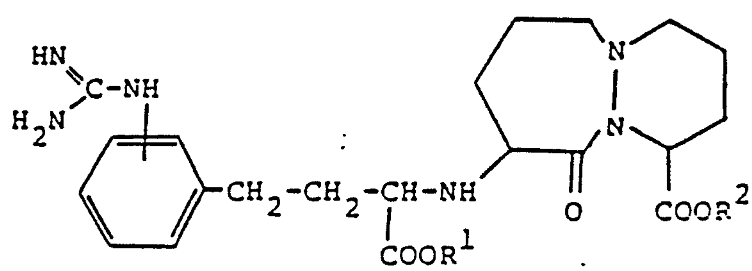

I

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl) bedeuten,
und pharmazeutisch verwendbare Säureadditionssalze davon besitzen antihypertensive Eigenschaften und können demnach in Form pharmazeutischer Präparate als Arzneimittel verwendet werden. Sie können nach bekannten Methoden hergestellt werden.

EP 0 271 795 A2

## Octahydro-10-oxo-6H-pyridazo[1,2-a][1,2]diazepin-Derivate, Zwischenprodukte und Verfahren zu deren Herstellung sowie diese enthaltende Arzneimittel

Die vorliegende Erfindung betrifft Pyridazodiazepinderivate, ein Verfahren zu deren Herstellung sowie Arzneimittel enthaltend diese Derivate.

Die erfindungsgemässen Pyridazodiazepinderivate sind Verbindungen der allgemeinen Formel

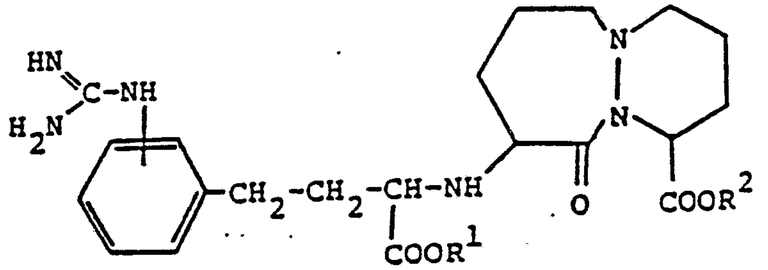

I

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl) bedeuten,
und pharmazeutisch verwendbare Salze davon.

Die Verbindungen der Formel I enthalten drei asymmetrische Kohlenstoffatome und können deshalb als optisch reine Diastereomere, als diastereomere Racemate oder als Mischungen verschiedener diastereomerer Racemate vorliegen. Die vorliegende Erfindung umfasst alle diese möglichen Formen. Die erfindungsgemässen Verbindungen weisen vorzugsweise an jedem asymmetrischen Kohlenstoffatom die (S)-Konfiguration auf.

Die in der vorliegenden Beschreibung verwendeten Ausdrücke "$C_1$-$C_4$-Alkyl" und "$C_1$-$C_{10}$-Alkyl" bedeuten geradkettige oder verzweigte Alkylreste mit der entsprechenden Anzahl Kohlenstoffatomen. Beispiele solcher Alkylgruppen sind Methyl, Aethyl, Propyl, Isopropyl, Butyl, tert.Butyl, n-Pentyl, n-Hexyl, n-Heptyl, n-Octyl, n-Nonyl, n-Decyl und dergleichen. Der Ausdruck "$C_2$-$C_6$-Alkanoyloxy" bedeutet eine Alkanoyloxygruppe, welche von einer geradkettigen oder verzweigten Alkancarbonsäure mit bis zu 6 Kohlenstoffatomen abgeleitet ist, z.B. Essigsäure, Propionsäure, Buttersäure, Pivalinsäure und dergleichen.

Die Verbindungen der Formel 1 bilden pharmazeutisch verwendbare Salze mit Säuren. Beispiele solcher Salze sind Mineralsäuresalze, wie Hydrohalogenide, z.B. Hydrobromide, Hydrochloride und dergleichen, Sulfate, Phosphate, Nitrate und dergleichen, und Salze mit organischen Säuren, wie Acetate, Maleate, Fumarate, Tartrate, Citrate, Salicylate, Methansulfonate, p-Toluolsulfonate und dergleichen. Die Verbindungen der Formel I, worin $R^1$ und/oder $R^2$ Wasserstoff bedeuten, bilden auch pharmazeutisch verwendbare Salze mit Basen. Beispiele solcher Salze sind Alkalimetallsalze, z.B. Natrium-und Kaliumsalze, Erdalkalimetallsalze, z.B. Calcium-und Magnesiumsalze, Ammoniumsalze und Salze mit organischen Aminen, z.B. Dicyclohexylaminsalze.

In der obigen Formel I bedeutet $R^1$ vorzugsweise Wasserstoff, $C_1$-$C_{10}$-Alkyl oder Adamantyl-($C_1$-$C_4$ -alkyl), besonders bevorzugt Wasserstoff, Aethyl, n-Decyl oder 1-Adamantyläthyl. $R^2$ bedeutet vorzugsweise Wasserstoff oder ($C_2$-$C_6$-Alkanoyloxy)-($C_1$-$C_4$-alkyl), besonders bevorzugt Wasserstoff oder Pivaloyloxymethyl. Die Guanidinogruppe $H_2N$-C(NH)-NH-befindet sich vorzugsweise in der para-Stellung des Phenylrings.

Aus dem obigen folgt, dass besonders bevorzugte Pyridazodiazepinderivate der Formel I solche sind, worin $R^1$ Wasserstoff, Aethyl, n-Decyl oder 1-Adamantyläthyl und $R^2$ Wasserstoff oder Pivaloyloxymethyl bedeuten und sich die Guanidinogruppe in der para-Stellung des Phenylrings befindet.

Die bevorzugtesten erfindungsgemässen Pyridazodiazepinderivate sind 9(S)-[1(S)-Carboxy-3-(4-guanidinophenyl)propylamino] -octahydro-10-oxo-6H-pyridazo[1,2-d][1,2]diazepin -1(S)-carbonsäure und dessen pharmazeutisch verwendbare Salze, besonders das Dihydrobromid.

Beispiele weiterer interessanter, erfindungsgemässer Pyridazodiazepinderivate sind:
9(S)-[1(S)-Aethoxycarbonyl-3-(4-guanidinophenyl)propylamino]-octahydro-10-oxo-6H-pyridazo[1,2-a][1,2]-diazepin-1(S)-carbonsäure,
9(S)-[1(S)-(n-Decyloxycarbonyl)-3-(4-guanidinophenyl)propylamino]-octahydro-10-oxo-6H-pyridazo[1,2-a]-[1,2]diazepin-1(S)-carbonsäure,
9(S)-[1(S)-[[2-(1-Adamantyl)äthoxy]carbonyl]-3-(4-guanidinophenyl)propylamino]-octahydro-10-oxo-6H-pyridazo[1,2-a][1,2]diazepin-1(S)-carbonsäure und

9(S)-[1(S)-Aethoxycarbonyl-3-(4-guanidinophenyl)propylamino]-octahydro-10-oxo-6H-pyridazo[1,2-a][1,2]-diazepin-1(S)-carbonsäure-pivaloyloxymethylester
sowie deren pharmazeutisch verwendbare Salze.

Die Verbindungen der Formel I und deren pharmazeutisch verwendbare Salze können erfindungsgemäss dadurch hergestellt werden, dass man

a) eine Verbindung der allgemeinen Formel

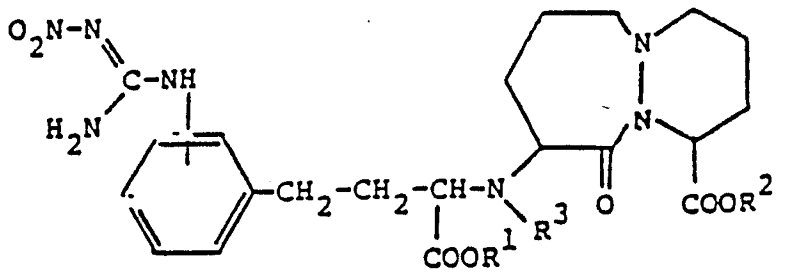

II

worin $R^1$ und $R^2$ die oben angegebene Bedeutung besitzen und $R^3$ Wasserstoff oder Benzyl bedeutet, in einem sauren Medium katalytisch hydriert und ein erhaltenes nicht-pharmazeutisch verwendbares Salz einer Verbindung der Formel I in eine Verbindung der Formel I überführt, oder

b) zur Herstellung einer Verbindung der Formel I, worin $R^1$ und/oder $R^2$ Wasserstoff bedeuten, eine Verbindung der Formel I, worin $R^1$ und/oder $R^2$ $C_1$-$C_{10}$-Alkyl bedeuten, mit einer Säure und/oder einer Base behandelt, und/oder

c) erwünschtenfalls, ein erhaltenes Gemisch von diastereomeren Racematen in die entsprechenden diastereomeren Racemate oder optisch reinen Diastereomeren auftrennt, und/oder

d) erwünschtenfalls, ein erhaltenes Racemat in die beiden optischen Antipoden auftrennt, und

e) erwünschtenfalls, eine erhaltene Verbindung der Formel I in ein pharazeutisch verwendbares Salz oder ein pharmazeutisch verwendbares Säureadditionssalz einer Verbindung der Formel I in eine Verbindung der Formel I überführt.

Die katalytische Hydrierung einer Verbindung der Formel II in einen sauren Medium gemäss Verfahrensvariante a) liefert ein Säureadditionssalz einer Verbindung der Formel I. Die katalytische Hydrierung kann in bekannter Weise durchgeführt werden, beispielsweise in Gegenwart eines Platin-oder Palladiumkatalysators, der auf einem inerten Trägermaterial aufgetragen sein kann. Palladium auf Kohle ist ein speziell geeigneter Katalysator. Die katalytische Hydrierung wird vorzugsweise in einem sauren Medium durchgeführt, welches ein pharmazeutisch verwendbares Säureadditionssalz einer Verbindung der Formel I liefert, vorzugsweise in einer geeigneten Alkancarbonsäure, wie Essigsäure, wässrige Salzsäure, wässrige Bromwasserstoffsäure oder ein Gemisch einer dieser wässrigen Säuren mit einer niederen Alkancarbonsäure. Gemäss einer weiteren Ausführungsform erhält man das saure Medium, zumindest partiell, durch Einsetzen eines Säureadditionssalzes einer Verbindung der Formel II. Es ist zweckmässig, die Hydrierung bei ungefähr Raumtemperatur und Atmosphärendruck durchzuführen.

Falls die katalytische Hydrierung ein nicht-pharmazeutisch verwendbares Säureadditionssalz einer Verbindung der Formel I liefert, wird dieses Salz in eine Verbindung der Formel I übergeführt. Diese Ueberführung kann dadurch erreicht werden, dass man das Salz in bekannter Weise mit einer Base behandelt.

Gemäss Verfahrensvariante b) wird eine Verbindung der Formel I, worin $R^1$ und/oder $R^2$ $C_1$-$C_{10}$-Alkyl bedeuten, in eine Verbindung der Formel I übergeführt, worin $R^1$ und/oder $R^2$ Wasserstoff bedeuten, indem man sie in Abhängigkeit von der Natur der $C_1$-$C_{10}$-Alkylgruppe mit einer Säure und/oder einer Base behandelt. Dieser Verfahrensschritt kann in bekannter Weise durchgeführt werden. Beispielsweise, falls $R^1$ und/oder $R^2$ tert.Butyl bedeuten, kann die Ueberführung durch Umsetzen mit einer geeigneten Säure, wie Bromwasserstoff in Essigsäure, oder Trifluor essigsäure, erfolgen, zweckmässigerweise bei ungefähr Raumtemperatur. Falls $R^1$ und/oder $R^2$ eine von tert.Butyl verschiedene $C_1$-$C_{10}$-Alkylgruppe bedeuten, kann die Ueberführung beispielsweise durch Umsetzen mit einer geeigneten Base, wie einem wässrigen äthanolischen Alkalimetallhydroxid, z.B. wässrigem äthanolischem Natriumhydroxid oder wässrigem äthanolischem Kaliumhydroxid, oder wässrigem Ammoniak bei einer Temperatur zwischen ungefähr Raumtemperatur und dem Siedepunkt des Reaktionsgemisches, vorzugsweise bei ungefähr Raumtemperatur, erfolgen.

Die Auftrennung von Mischungen diastereomerer Racemate in die diastereomeren Racemate oder optisch reinen Diastereomeren gemäss Verfahrensvariante c) erfolgt nach an sich bekannten Methoden, beispielsweise durch Chromatographie, z.B. an Silicagel, unter Verwendung eines geeigneten

Lösungsmittelsystems, z.B. Aethylacetat/n-Hexan, Toluol/Aethylacetat und dergleichen.

Die Auftrennung eines Racemates in die beiden optischen Antipoden gemäss Verfahrensvariante d) kann ebenfalls in bekannter Weise durchgeführt werden, beispielsweise durch Umsetzen mit einer geeigneten optisch aktiven Säure oder Base in Abhängigkeit von der Natur des aufzutrennenden Racemates, Abtrennen des erhaltenen optisch aktiven Salzes, z.B. durch fraktionierte Kristallisation, und nötigenfalls Freisetzen der optisch einheitlichen Verbindungen aus diesen Salzen nach bekannten Methoden.

Die Ueberführung einer Verbindung der Formel I in ein pharmazeutisch verwendbares Salz gemäss Verfahrensvariante e) kann in bekannter Weise durch Umsetzen mit einer geeigneten Säure oder, falls $R^1$ und/oder $R^2$ in der Verbindung der Formel I Wasserstoff bedeuten, mit einer geeigneten Base erfolgen. Die Ueberführung eines pharmazeutisch verwendbaren Säureadditionssalzes einer Verbindung der Formel I in eine Verbindung der Formel I, ebenfalls gemäss Verfahrensvariante e), kann in bekannter Weise durch Umsetzen mit einer geeigneten Base erfolgen.

Die als Ausgangsstoffe eingesetzten Verbindungen der Formel II können hergestellt werden, indem man zunächst beispielsweise eine Verbindung der allgemeinen Formel

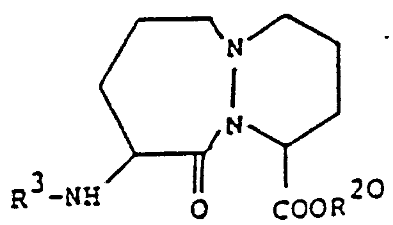

III

worin $R^3$ Wasserstoff oder Benzyl und $R^{20}$ $C_1$-$C_{10}$-Alkyl bedeuten, mit einer Verbindung der allgemeinen Formel

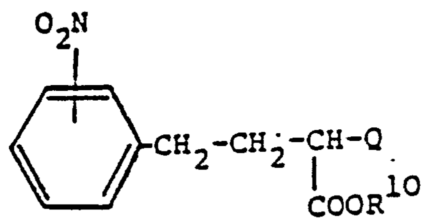

IV

worin $R^{10}$ $C_1$-$C_{10}$-Alkyl und Q eine Abgangsgruppe bedeuten, zu einer Verbindung der allgemeinen Formel

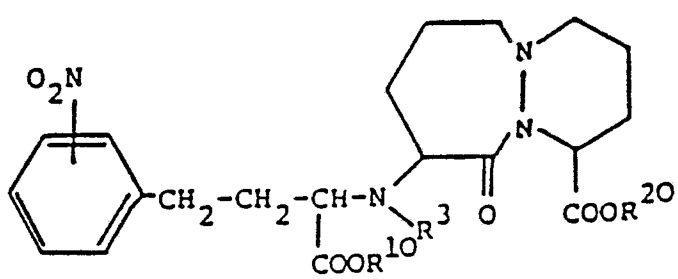

V

worin $R^3$, $R^{10}$ und $R^{20}$ die oben angegebene Bedeutung besitzen, umsetzt und erwünschtenfalls die erhaltene Verbindung der Formel V mit einer Säure und/oder einer Base zu einer Verbindung der allgemeinen Formel

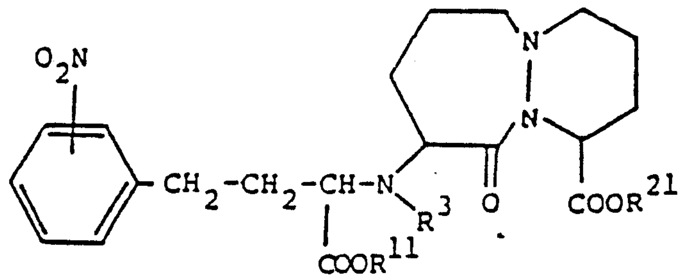

VI

worin $R^3$ die oben angegebene Bedeutung besitzt und $R^{11}$ und/oder $R^{21}$ Wasserstoff bedeuten, umsetzt und, ebenfalls erwünschtenfalls, eine Verbindung der Formel VI zu einer Verbindung der allgemeinen Formel

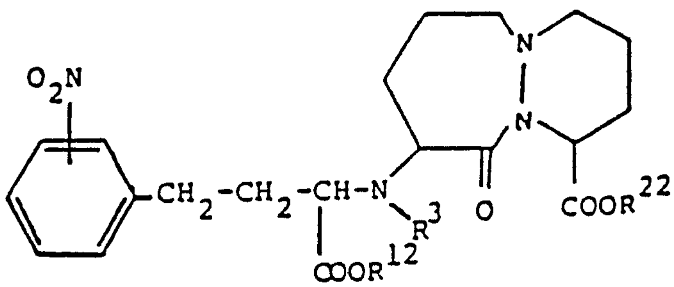

VII

worin $R^3$ die oben angegebene Bedeutung besitzt und $R^{12}$ und/oder $R^{22}$ $C_1$-$C_{10}$-Alkyl (welches von in Formel V durch $R^{10}$ und $R^{20}$ bezeichnetem $C_1$-$C_{10}$-Alkyl verschieden ist), Adamantyl-($C_1$-$C_4$-alkyl) oder ($C_2$-$C_6$-Alkanoyloxy)-($C_1$-$C_4$-alkyl) bedeuten,
verestert.

Die Abgangsgruppe Q in einer Verbindung der Formel IV kann jede konventionelle Abgangsgruppe sein, beispielsweise, ein Halogenatom, wie ein Bromatom, oder eine Sulfonatgruppe der Formel $-O-SO_2Y$, worin Y Methyl, Trifluormethyl, p-Tolyl, 4-Nitrophenyl und dergleichen bedeutet.

Die Umsetzung einer Verbindung der Formel III mit einer Verbindung der Formel IV unter Bildung einer Verbindung der Formel V kann in bekannter Weise erfolgen, zweckmässigerweise in Gegenwart eines inerten organischen Lösungsmittels, wie Acetonitril, Dimethylsulfoxid, Dimethylformamid und dergleichen, und in Gegenwart eines säurebindenden Mittels, wie eines Alkalimetallcarbonates, z.B. Natriumcarbonat, eines basischen Ionenaustauscherharzes oder, vorzugsweise, einer tertiären organischen Base, z.B. Triäthylamin. Die Umsetzung kann bei einer Temperatur zwischen ungefähr 0°C und dem Siedepunkt des Reaktionsgemisches durchgeführt werden.

Die Behandlung einer Verbindung der Formel V mit einer Säure und/oder einer Base zur Bildung einer Verbindung der Formel VI kann in bekannter Weise durchgeführt werden. Die speziellen Reaktionsbedingungen, welche notwendig sind, hängen selbstverständlich von der Natur der $C_1$-$C_{10}$-Alkylgruppen ab, die in der Verbindung der Formel V vorhanden sind. Beispielsweise, wenn $R^{10}$ und/oder $R^{20}$ tert.Butyl bedeuten, kann die Umsetzung unter Verwendung einer geeigneten Säure, wie wasserfreie Trifluoressigsäure, zweckmässigerweise bei ungefähr Raumtemperatur, oder Bromwasserstoff in Essigsäure, ebenfalls zweckmässigerweise bei ungefähr Raumtemperatur, erfolgen. Wiederum beispielsweise kann die Umsetzung unter Verwendung einer geeigneten Base, wie einem wässrigen äthanolischen Alkalimetallhydroxid, z.B. wässrigem äthanolischem Natriumhydroxid oder wässrigem äthanolischem Kaliumhydroxid, oder wässrigem Ammoniak bei einer Temperatur zwischen ungefähr Raumtemperatur und dem Siedepunkt des Reaktionsgemisches, vorzugsweise bei ungefähr Raumtemperatur, durchgeführt werden, falls $R^{10}$ und/oder $R^{20}$ eine von tert.Butyl verschiedene $C_1$-$C_{10}$-Alkylgruppe bedeuten.

Die Veresterung einer Verbindung der Formel VI zu einer Verbindung der Formel VII kann in bekannter Weise durchgeführt werden. Beispielsweise kann eine Verbindung der Formel VI durch Umsetzen mit einem geeigneten Alkanol oder einem zweckmässig substituierten Alkanol in Gegenwart von N,N'-Dicyclohexylcarbodiimid verestert werden. Eine Verbindung der Formel VI, worin $R^3$ Benzyl bedeutet, kann -wiederum beispielsweise - verestert werden, indem man sie mit einem geeigneten Alkylbromid oder einem zweckmässig substituierten Alkylbromid in Gegenwart einer starken Base, z.B. Kaliumhydroxid, oder Cäsiumcarbonat, umsetzt.

Danach wird eine Verbindung der Formel V, VI oder VII zu einer Verbindung der allgemeinen Formel

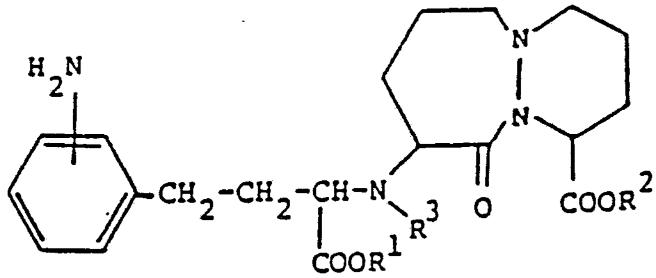

VIII

worin $R^1$, $R^2$ und $R^3$ die oben angegebene Bedeutung besitzen, reduziert.

Die Reduktion einer Verbindung der Formel V, VI oder VII zu einer Verbindung der Formel VIII kann in bekannter Weise durchgeführt werden. Beispielsweise kann die Reduktion mittels katalytischer Hydrierung in Gegenwart eines Edelmetallkatalysators, wie Platin oder Palladium, welches auf einem inerten Trägermaterial aufgetragen sein kann, erfolgen. Palladium auf Kohle kann für den vorliegenden Zweck als besonders geeigneter Katalysator genannt werden. Diese katalytische Hydrierung wird vorteilhafterweise in einem inerten organischen Lösungsmittel, insbesondere in einem Alkanol, wie Methanol, Aethanol und dergleichen, bei Raumtemperatur und Atmosphärendruck durchgeführt. Die Reduktion kann man beispielsweise aber auch unter Verwendung von Zink/Essisäure nach bekannten Methoden durchführen.

Eine Verbindung der Formel VIII, worin $R^1$ und/oder $R^2$ $C_1$-$C_{10}$-Alkyl bedeuten, kann erwünschtenfalls in eine Verbindung der Formel VIII übergeführt werden, worin $R^1$ und/oder $R^2$ Wasserstoff bedeuten, indem man diese mit einer Säure und/oder einer Base behandelt. Diese Behandlung kann unter den gleichen Reaktionsbedingungen erfolgen, wie sie weiter oben im Zusammenhang mit der Ueberführung einer Verbindung der Formel V in eine Verbindung der Formel VI angegeben sind.

Eine Verbindung der Formel VIII wird danach mit 1-Nitroguanyl-3,5-dimethylpyrazol zu einer Verbindung der Formel II umgesetzt.

Die Umsetzung einer Verbindung der Formel VIII mit 1-Nitroguanyl-3,5-dimethylpyrazol kann nach bekannten Methoden erfolgen. Die Umsetzung erfolgt zweckmässigerweise in Gegenwart eines inerten organischen Lösungsmittels, insbesondere eines Alkanols, wie Methanol, Aethanol und dergleichen, und bei erhöhter Temperatur, zweckmässigerweise bei der Rückflusstemperatur des Reaktionsgemisches.

Eine Verbindung der Formel II, worin $R^1$ und/oder $R^2$ $C_1$-$C_{10}$-Alkyl bedeuten, kann erwünschtenfalls in eine Verbindung der Formel II übergeführt werden, worin $R^1$ und/oder $R^2$ Wasserstoff bedeuten, indem man diese mit einer Säure und/oder einer Base behandelt. Diese Behandlung kann in gleicher Weise durchgeführt werden, wie weiter oben im Zusammenhang mit der Ueberführung einer Verbindung der Formel V in eine Verbindung der Formel VI beschrieben.

Die Verbindungen der obigen Formeln II, V, VI, VII und VIII, welche durch die nachstehende allgemeine Formel

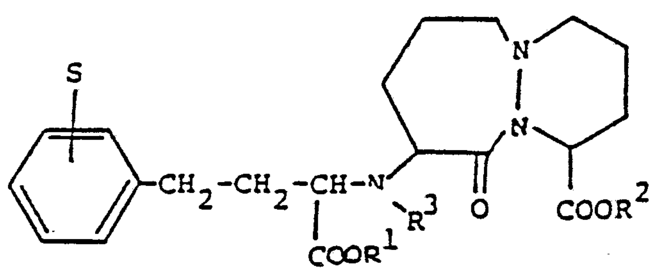

IX

worin $R^1$, $R^2$ und $R^3$ die oben angegebene Bedeutung besitzen und S Nitro, Amino oder 2-Nitroguanidino bedeutet,

sind neu und ebenfalls Gegenstand vorliegender Erfindung.

Die Verbindungen der Formel III, worin $R^3$ Wasserstoff bedeutet, sind bekannt oder Analoge bekannter Verbindungen. Die Verbindungen der Formel III, worin $R^3$ Benzyl bedeutet, können durch reduktive Benzylierung einer Verbindung der Formel III erhalten werden, worin $R^3$ Wasserstoff bedeutet.

Die Verbindungen der Formel IV sind bekannte Verbindungen oder Analoge der bekannten Verbindungen.

Die erfindungsgemässen Pyridazodiazepinderivate hemmen das Angiotensin umwandelnde Enzym (ACE), welches für die Umwandlung von Angiotensin I in Angiotensin II verantwortlich ist, und sind deshalb wertvolle Antihypertensiva. Darüberhinaus besitzen sie eine überraschend langanhaltende Wirkungsdauer.

Die Aktivität der vorliegenden Pyridazodiazepinderivate, das Angiotensin umwandelnde Enzym in vitro zu hemmen, kann durch den folgenden Test bestimmt werden.

Die verwendete Methode basiert auf einer Methode von R.A.S. Santos, E.M. Kreiger und L.J. Greene, Hypertension (1985), 7, 244-252. Angiotensin umwandelndes Enzym (Kaninchenlunge) wird in Abwesenheit oder Gegenwart verschiedener Konzentrationen der Testverbindungen in 0,1M Kaliumphosphatpuffer (pH 7,5), welcher 30 mMol Natriumchlorid enthält, während 90 Minuten bei 37°C inkubiert. [Falls die Testsubstanz ein Ester ist, wird dieser mittels Schweineleberesterase vor Ausführung des Tests gespalten.] Die Reaktion wird initiiert, indem man Angiotensin I bis zu einer Konzentration von 250 µMol zugibt. Das Schlussvolumen der Reaktionsmischung beträgt 0,25 ml. Nachdem das Reaktionsgemisch während 30 Minuten bei 37°C gehalten wurde, wird die Reaktion durch Zugabe von 1,45 ml 0,3M Natriumhydroxidlösung abgebrochen. 100 ml einer 0,2%igen (Gewicht/Volumen) Lösung von o-Phthaldialdehyd in Methanol werden zugegeben, und nach 10 Minuten wird die Lösung mit 200 µl 3M Salzsäure angesäuert. Die erhaltene Lösung wird dann fluoreszenzspektrometrisch untersucht, indem man bei einer Wellenlänge von 365 nm anregt und die Emission bei 500 nm misst und den $IC_{50}$-Wert berechnet. Der $IC_{50}$-Wert ist diejenige Konzentration einer Testverbindung, welche die Umwandlung von Angiotensin I in Angiotensin II um 50% reduziert.

Im oben beschriebenen Test besitzt 9(S)-[1(S)-Carboxy-3-(4-guanidinophenyl)propylamino] -octahydro-10-oxo-6H-pyridazo[1,2-a][1,2]diazepin -1(S)-carbonsäure-dihydrobromid einen $IC_{50}$-Wert von 0,6 nMol.

Die erfindungsgemässen Pyridazodiazepinderivate können in Form pharmazeutischer Präparate, welche diese Verbindungen zusammen mit geeigneten pharmazeutischen Trägerstoffen enthalten, als Arzneimittel verwendet werden. Als Trägerstoffe kommen dabei für die enterale, beispielsweise orale, oder parenterale Verabreichung geeignete, organische oder anorganische Trägerstoffe in Frage, beispielsweise Wasser, Gelatine, Gummi arabicum, Lactose, Stärke, Magnesiumstearat, Talk, pflanzliche Oele, Polyalkylenglykole, Vaseline und dergleichen. Die pharmazeutischen Präparate können in fester Form, z.B. als Tabletten, Dragées, Suppositorien oder Kapseln, oder in flüssiger Form, z.B. als Lösungen, Suspensionen oder Emulsionen, verarbeitet werden. Die pharmazeutischen Präparate können üblichen pharmazeutischen Behandlungen, wie Sterilisieren, unterworfen werden und/oder Zusatzstoffe, wie Konservierungsmittel, Stabilisierungsmittel, Netzmittel oder Emulgiermittel, Salze zum Variieren des osmotischen Druckes oder Puffer enthalten. Die pharmazeutischen Präparate können auch noch andere therapeutisch wertvolle Verbindungen enthalten.

Die erfindungsgemässen Pyridazodiazepinderivate können Erwachsenen in täglichen Dosen von etwa 0,1 bis 100 mg, vorzugsweise etwa 1-50 mg, pro Kilogramm Körpergewicht verabreicht werden. Die tägliche Dosis kann in einer einzigen Dosis oder in Teildosen verabreicht werden. Es sei an dieser Stelle betont, dass die oben genannten Dosierungsbereiche nur als Beispiele angegeben sind und dass sie nach oben oder unten variiert werden können in Abhängigkeit von Faktoren, wie den spezifischen Eigenschaften des zu verabreichenden Derivates, der Art der Verabreichung, der Schwere der zu behandelnden Krankheit und dem Zustand des Patienten, wie er vom behandelnden Arzt festgestellt wird.

Die Verbindungen der obigen Formel IX, worin $R^3$ Wasserstoff bedeutet, hemmen ebenfalls das Angiotensin umwandelnde Enzym und können als Antihypertensiva verwendet werden.

Die folgenden Beispiele veranschaulichen die vorliegende Erfindung.

Beispiel 1

Eine Lösung von 0,70 g 9(S)-[1(S)-Carboxy-3-[4 -(2-nitroguanidino)phenyl]propylamino]-octahydro-10 -oxo-6H-pyridazo[1,2-a][1,2]diazepin -1(S)-carbonsäure-hydrobromid in einem Gemisch von 16 ml Essigsäure, 4 ml Wasser und 4 Tropfen Bromwasserstoffsäure wird bei Raumtemperatur und Atmosphärendruck während 72 Stunden über 10% Palladium auf Kohle hydriert. Der Katalysator wird abfiltriert, und das Filtrat eingedampft. Das zurückbleibende Oel wird in 50 ml Wasser gelöst, und die Lösung lyophilisiert, wobei man 0,735 g 9(S)-[1(S)-Carboxy-3-(4-guanidinophenyl)propylamino] -octahydro-10-oxo-6H-pyridazo[1,2-a][1,2]diazepin -1(S)-carbonsäure-dihydrobromid als weisslichen amorphen Festkörper erhält.

NMR: $\delta_H$ ($CD_3OD$, 300 MHz): 1.44 (1H, m), 1.68-1.98 (4H, m), 2.04-2.36 (4H, m), 2.42 (1H, m), 2.63 (1H, m), 2.82-2.98 (2H, m), 3.04 (1H, breit, d), 3.17 (1H, m), 3.48 (1H, m), 4.01 (1H, t), ungefähr 4.90 (2H, teilweise

vom Wasser-Signal verdeckt), 7.25 (2H, d) und 7.41 (2H, d).

MS: m/e 447 (20% [M + H] ) und 211 (100).

Das als Ausgangsmaterial eingesetzte 9(S)-[1(S)-Carboxy-3-[4-(2-nitroguanidino)phenyl]propylamino] -octahydro-10-oxo-6H-pyridazo[1,2-a][1,2]diazepin -1(S)-carbonsäure-hydrobromid wurde wie folgt hergestellt:

(A)(i) Eine Lösung von 0,566 g tert.Butyl 9(S)-amino-octahydro-10-oxo-6H-pyridazo[1,2-a][1,2]diazepin -1(S)-carboxylat, 0.688 g 2 (R,S)-Brom-4-(4-nitrophenyl)butansäure-tert.butylester und 0.202 g Triäthylamin in 12 ml Acetonitril wird während 16 Stunden auf 80°C erhitzt. Das Lösungsmittel wird danach abgedampft, und der Rückstand zwischen Wasser und Methylenchlorid verteilt. Die organische Lösung wird eingedampft, und das verbleibende Oel an Silica gel chromatographiert. Eluieren mit Toluol/Aethylacetat (1:1) liefert zunächst 0.195 g tert.Butyl 9(S)-[1-(R)-tert.-butoxycarbonyl-3-(4-nitrophenyl)propylamino] -octahydro-10-oxo-6H-pyridazo[1,2-a][1,2]diazepin -1(S)-carboxylat als gelbes Oel und danach 0,252 g tert.Butyl 9(S)-[1-(S)-tert.-butoxycarbonyl-3-(4-nitrophenyl)propylamino] -octahydro-10-oxo-6H-pyridazo[1,2-a][1,2]diazepin -1(S)-carboxylat als gelbes Oel.

Analyse für $C_{28}H_{42}N_4O_7$:
Berechnet: C: 61.5; H: 7.70; N: 10.25%
Gefunden: C: 61.5; H: 7.75; N: 10.20%.

A(ii)(a) Eine Lösung von 18 g 2(R)-Hydroxy-4-phenylbutansäure in 200 ml Methylenchlorid, enthaltend 20.5 g Triäthylamin und 250 mg 4-(Dimethylamino)pyridin wird bei Raumtemperatur gerührt und tropfenweise mit 12 g Essigsäureanhydrid versetzt. Das erhaltene Reaktionsgemisch wird während 3 weiteren Stunden gerührt und dann durch Zugabe von 125 ml 2N Schwefelsäure angesäuert. Die organische Phase wird abgetrennt und mit zwei 100 ml Portionen 2N Schwefelsäure, dann mit 100 ml Wasser und schliesslich mit 100 ml gesättigter Natriumchloridlösung gewaschen, über wasserfreiem Magnesiumsulfat getrocknet und unter vermindertem Druck eingedampft, wobei man 22 g 2(R)-Acetoxy-4-phenylbutansäure als farbloses viskoses Oel erhält,
$[\alpha]^{20}_{436} = +13.7°$ (c = 1 in Aethanol).

(b) Eine Lösung von 22 g 2(R)-Acetoxy-4-phenylbutansäure in 10 ml Eisessig wird unter Rühren tropfenweise zu einem auf -5°C gekühlten Nitrierungsgemisch gegeben, welches seinerseits hergestellt wird, indem man 12,6 g rauchende 95%ige Salpetersäure tropfenweise zu 41 g Essigsäure gibt, wobei man die Innentemperatur während der ganzen Zeit mit einem Kühlbad (-10°C bis -5°C) bei etwa 5°C hält und schliesslich die Temperatur des Nitrierungsgemisches auf -5°C fallen lässt. Das erhaltene Gemisch wird dann noch während 2 weiteren Stunden bei -5°C gerührt und dann in ungefähr 200 ml Eiswasser gegossen und während mehreren Stunden gerührt. Das Gemisch wird dann mit dreimal je 100 ml Diäthyläther extrahiert, und die vereinigten organischen Extrakte dreimal mit je 100 ml Wasser und mit 100 ml gesättigter Natriumchloridlösung gewaschen, über wasserfreiem Magnesiumsulfat getrocknet und unter vermindertem Druck eingedampft. Dabei erhält man 27 g eines schwach gelben Oels, welches gemäss $^1$H-NMR-spektroskopischer Bestimmung aus einem ungefähr 1:2-Gemisch von 2(R)-Acetoxy-4-(2-nitrophenyl)-butansäure und 2(R)-Acetoxy-4-(4-nitrophenyl)butansäure besteht. Dieses Oel wird in 50 ml warmem Toluol gelöst und zur Kristallisation einige Stunden bei 0-5°C stehen gelassen. Auf diese Weise erhält man 9.6 g 2(R)-Acetoxy-4-(4-nitrophenyl)butansäure als farblosen kristallinen Festkörper vom Schmelzpunkt 108-109°C.

(c) 8.0 g 2(R)-Acetoxy-4-(4-nitrophenyl)butansäure werden unter Rühren portionsweise zu einer Lösung von 4 g tert.Butanol, 7 g N,N'-Dicyclohexylcarbodiimid end 450 mg 4-Pyrrolidinpyridin in 200 ml Methylenchlorid gegeben, und das Gemisch 16 Stunden bei 0°C gerührt. Das Gemisch wird filtriert, und das Filtrat nacheinander mit 100 ml Wasser, zweimal 100 ml 2N Essigsäure, 100 ml Wasser, zweimal 100 ml gesättigter Natriumbicarbonatlösung und 100 ml gesättigter Natriumchloridlösung gewaschen, über wasserfreiem Magnesiumsulfat getrocknet und unter vermindertem Druck eingedampft, wobei man 10 g eines schwach gelben halbfesten Rückstands erhält. Dieses Rückstand wird mit einer geringen Menge Diäthyläther angerieben und filtriert, und das Filtrat durch eine Silicagelsäule mit Diäthyläther/n-Hexan (1:1) filtriert, wobei man 9 g 2(R)-Acetoxy-4-(4-nitrophenyl)butansäure-tert.butylester als schwach gelbes Oel erhält, MS: m/e 324 (10%, [M + H] ) und 268 (100).

(d) 8.0 g 2(R)-Acetoxy-4-(4-nitrophenyl)butansäure-tert.butylester werden in 200 ml bei 0°C mit Ammoniak gesättigtem Methanol gelöst, und das Gemisch 16 Stunden gerührt. Eindampfen des Reaktionsgemisches unter vermindertem Druck liefert 7 g eines fast farblosen Oels, welches in einer gleichen Menge eines 1:1-Gemisches von Diäthyläther und n-Hexan gelöst und über eine Silicagelsäule unter Verwendung des gleichen Lösungsmittelgemisches filtriert wird, wobei man 6 g 2(R)-Hydroxy-4-(4-nitrophenyl)-butansäure-tert.butylester als farblosen kristallinen Festkörper erhält, Schmelzpunkt 41-42°C.

(e) Eine Lösung von 0,4 ml trockenem Pyridin in 20 ml über Molekularsieb getrocknetem Methylenchlorid wird unter Rühren und wasserfreien Bedingungen auf -20°C gekühlt und dann tropfenweise mit 1.4 g Trifluormethansulfonsäureanhydrid versetzt. Nach 5 Minuten wird eine Lösung von 1,4 g 2(R)-Hydroxy-4-(4-nitrophenyl)butansäure-tert.butylester in 5 ml trockenem Methylenchlorid zugegeben, und das erhaltene Reaktionsgemisch 16 Stunden bei 0°C gerührt. Das Gemisch wird danach über eine Silicagelsäule filtriert, und die Säule mit zwei 10 ml Portionen von trockenem Methylenchlorid gewaschen. Die vereinigten Filtrate, enthaltend 2(R)-Trifluormethansulfonyloxy-4-(4-nitrophenyl)butansäure -tert.butylester, werden mit 0.5 g trockenem Triäthylamin und 1.4 g tert.Butyl 9(S)-amino-octahydro-10-oxo-6H -pyridazo[1,2-a][1,2]diazepin-1(S)-carboxylat versetzt, und das Reaktionsgemisch 1 Stunde bei Raumtemperatur gerührt. Das Gemisch wird dann nacheinander mit 25 ml Wasser, 25 ml gesättigter Natriumbicarbonatlösung und 25 ml gesättigter Natriumchloridlösung gewaschen, über wasserfreiem Magnesiumsulfat getrocknet und unter vermindertem Druck zu 3 g eines gelben Oels eingedampft. Chromatographie an Silicagel unter Verwendung von Diäthyläther als Eluierungsmittel liefert 1.7 g (60%) tert.Butyl 9(S)-[1(S)-tert.butoxycarbonyl-3-(4-nitrophenyl)propylamino] -octahydro-10-oxo-6H-pyridazo[1,2-a][1,2]diazepin -1(S)-carboxylat als gelbes Oel, welches mit der Verbindung identisch ist, wie sie nach dem in Paragraph (A)(i) beschriebenen Verfahren erhalten wurde.

(A)(iii)(a) Eine Lösung von 2.8 g 2(R)-Hydroxy-4-(4-nitrophenyl)butansäure-tert.butylester (hergestellt wie in Paragraph (A)(ii)(d) beschrieben) und 2 ml trockenem Triäthylamin in 20 ml trockenem Methylenchlorid wird unter Rühren tropfenweise zu einer auf 0°C abgekühlten Lösung von 2.2 g 4-Nitrobenzolsulfonylchlorid in 40 ml trockenem Methylenchlorid gegeben, und das Gemisch 16 Stunden bei 0°C gerührt. Die erhaltene Lösung wird nacheinander mit 50 ml Wasser, dreimal 50 ml 1N Schwefelsäure, 50 ml Wasser, zweimal 50 ml gesättigter Natriumbicarbonatlösung und 50 ml gesättigter Natriumchloridlösung gewaschen, über wasserfreiem Magnesiumsulfat getrocknet und unter vermindertem Druck eingedampft, wobei man 4,7 g eines schwach gelben Gummis erhält. Anreiben dieses Gummis in 10 ml Diäthyläther liefert 3.8 g (82%) 4-(4-Nitrophenyl)-2(R)-[(4-nitrophenyl)sulfonyloxy]butansäure -tert.-butylester als schwach gelben kristallinen Festkörper, Schmelzpunkt 97-98°C.

(b) 2 g tert.Butyl 9(S)-amino-octahydro-10-oxo-6H-pyridazo[1,2-a][1,2]diazepin -1(S)-carboxylat werden zu einer Lösung von 3.3 g 4-(4-Nitrophenyl)-2(R)-[(4-nitrophenyl)sulfonyloxy]butansäure -tert.butylester und 1 ml trockenem Triäthylamin in 50 ml trockenem Acetonitril gegeben, und das Reaktionsgemisch 20 Stunden zum Rückfluss erhitzt. Die erhaltene Lösung wird unter vermindertem Druck eingedampft, und der ölige Rückstand in 50 ml Methylenchlorid gelöst und nacheinander mit zweimal 50 ml Wasser, 50 ml gesättigter Natriumbicarbonatlösung und 50 ml gesättigter Natriumchloridlösung gewaschen, über wasserfreiem Magnesiumsulfat getrocknet und unter vermindertem Druck eingedampft, wobei man 4,g eines gelben Oels erhält. Chromatographie an Silicagel unter Verwendung von Diäthyläther als Eluierungsmittel liefert 2.7 g (70%) tert.Butyl 9(S)-[1-(S))-tert.butoxycarbonyl-3 -(4-nitrophenyl)propylamino]octahydro-10-oxo -6H-pyridazo[1,2-a][1,2]diazepin-1(S)-carboxylat als gelbes Oel, welches mit der Verbindung identisch ist, wie sie nach dem in Paragraph (A)(i) beschriebenen Verfahren erhalten wurde.

(B) Eine Lösung von 4.63 g tert.Butyl 9(S)-[1(S)-tert.-butoxycarbonyl-3-(4-nitrophenyl)propylamino] -octahydro-10-oxo-6H-pyridazo[1,2-a][1,2]diazepin -1(S)-carboxylat in 100 ml Aethanol wird über 10% Palladium auf Kohle 3 Stunden bei Raumtemperatur und Atmosphärendruck hydriert. Der Katalysator wird abfiltriert, und das Filtrat eingedampft. Das erhaltene gelbe Oel wird in 40 ml Aethanol gelöst, 1,72 g 1-Nitroguanyl-3,5-dimethylpyrazol werden zugegeben, und die Lösung 48 Stunden zum Rückfluss erhitzt. Das Lösungsmittel wird danach abgedampft, und das zurückbleibende Oel an Silicagel chromatographiert. Eluieren mit 3% Methanol in Diäthyläther und nachfolgende Kristallisation aus Aethylacetat/n-Hexan liefert 1.66 g tert.Butyl 9(S)-[1(S)-tert.-butoxycarbonyl-3-[4 -(2-nitroguanidino)phenyl]propylamino]-octahydro-10-oxo -6H-pyridazo[1,2-a][1,2]diazepin-1(S)-carboxylat als weisslichen Festkörper.

Analyse für $C_{29}H_{45}N_7O_7$:
Berechnet: C: 57.7; H: 7.5; N: 16.20%
Gefunden: C: 57.7; H: 7.5; N: 15.95%.

(C)(i) Eine Lösung von 0.182 g tert.Butyl 9(S)-[1(S)-tert.butoxycarbonyl-3 -[4-(2-nitroguanidino)phenyl]-propylamino]-octahydro-10-oxo -6H-pyridazo[1,2-a][1,2]diazepin-1(S)-carboxylat in 0.6 ml Essigsäure wird mit 1.8 ml 45%igem Bromwasserstoff in Essigsäure versetzt, und die Reaktionslösung 1,25 Stunden bei 20°C gerührt. Die Lösung wird danach in 100 ml wasserfreien Diäthyläther gegossen, 2 Stunden gerührt und dann filtriert, wobei man 0.17 g 9(S)-[1(S)-Carboxy-3 -[4-(2-nitroguanidino)phenyl]propylamino]-octahydro-10-oxo -6H-pyridazo[1,2-a][1,2]diazepin-1(S)-carbonsäure-hydrobromid als weissen Festkörper erhält.

NMR: $\delta_H$ ($CD_3OD$, 300 MHz): 1.42 (1H, m), 1.68-1.96 (4H, m), 2.09-2.35 (4H, m), 2.38 (1H, m), 2.60 (1H, m), 2.78-2.96 (2H, m), 3.04 (1H, breit, d), 3.15 (1H, m), 3.48 (1H, m), 4.01 (1H, m), 4.84 (1H, t), ungefähr 4.91 (1H, teilweise vom Wasser-Signal verdeckt), 7.29 (2H, d) und 7.34 (2H, d).
MS: m/e 492 (2% [M + H] ) und 211 (100).

Beispiel 2

Eine Lösung von 6 g tert.Butyl 9(S)-[1(S)-tert.butoxycarbonyl-3-[4 -(2-nitroguanidino)phenyl]-propylamino]-octahydro-10-oxo -6H-pyridazo[1,2-a][1,2]diazepin-1(S)-carboxylat in 60 ml Trifluoressigsäure wird 6 Stunden bei Raumtemperatur gerührt und danach unter vermindertem Druck eingeengt, wobei man einen gummiartigen Rückstand von 9(S)-[1(S)-Carboxy-3-[4-(2 -nitroguanidino]phenyl]propylamino]-octahydro-10-oxo -6H-pyridazo[1,2-a][1,2]diazepin-1(S)-carbonsäure-trifluoracetat zusammen mit überschüssiger Trifluoressigsäure erhält. Dieser gummiartige Rückstand wird in einem Gemisch von 20 ml Isopropanol und 100 ml destilliertem Wasser gelöst, und die erhaltene klare Lösung über 10% Palladium auf Kohle 24 Stunden bei Raumtemperatur und Atmosphärendruck hydriert. Der Katalysator wird dann abfiltriert, und das Filtrat danach bis zur neutralen Reaktion (pH 7) tropfenweise mit Ammoniumhydroxidlösung versetzt. Dann wird das Reaktionsgemisch unter vermindertem Druck auf ein Volumen von ungefähr 50 ml eingeengt und dann mehrer Stunden bis zur beendeten Kristallisation bei Raumtemperatur stehengelassen. Die Kristalle werden abfiltriert, nacheinander mit minimalen Volumina von destilliertem Wasser, Aethanol und Diäthyläther gewaschen und schliesslich an der Luft getrocknet, wobei man 4 g 9(S)-[1(S)-Carboxy-3-(4-guanidinophenyl)propylamino] -octahydro-10-oxo-6H-pyridazo[1,2-a][1,2]diazepin -1(S)-carbonsäure-pentahydrat als weissen kristallinen Festkörper erhält, Schmelzpunkt 230-232°C (Zers.).

Beispiel 3

Eine Lösung von 0,45 g 9(S)-[1(S)-Aethoxycarbonyl-3-[4 -(2-nitroguanidino)phenyl]propylamino]-octahydro-10-oxo -6H-pyridazo-[1,2-a][1,2]diazepin-1(S)-carbonsäure-hydrobromid in einem Gemisch von 20 ml Essigsäure, 5 ml Wasser und 5 Tropfen Bromwasserstoffsäure wird über 10% Palladium auf Kohle bei Raumtemperatur und Atmosphärendruck 132 Stunden hydriert. Der Katalysator wird abfiltriert, und das Filtrat eingedampft. Der Rückstand wird mit 200 ml wasserfreiem Diäthyläther 16 Stunden gerührt, und das Gemisch danach filtriert, wobei man 0.57 g 9(S)-[1(S)-Aethoxycarbonyl-3 -(4-guanidinophenyl)propylamino]-octahydro-10-oxo -6H-pyridazo[1,2-a][1,2]diazepin -1(S)-carbonsäure-dihydrobromid als amorphen weisslichen Festkörper erhält.
NMR: $\delta_H$ ($CD_3OD$, 300 MHz): 1.35 (3H, t), 1.44 (1H, m), 1.69-1.94 (4H, m), 2.10-2.35 (4H, m), 2.40 (1H, breit, d), 2.61 (1H, m), 2.83 (1H, m), 2.92 (1H, m), 3.04 (1H, breit, d), 3.18 (1H, m), 3.49 (1H, m), 4.11 (1H, t), 4.35 (2H, m), 4.86-4.95 (2H, teilweise vom Wasser-Signal verdeckt), 7.26 (2H, d) und 7.40 (2H, d).
MS: m/e 475 (20%, [M + H] ) und 211 (100).

Das als Ausgangsmaterial eingesetzte 9(S)-[1(S)-Aethoxycarbonyl-3-[4 -(2-nitroguanidino)phenyl]-propylamino]-octahydro-10-oxo -6H-pyridazo[1,2-a][1,2]diazepin-1(S)-carbonsäure-hydrobromid wurde wie folgt hergestellt:

(A) Eine Lösung von 2.83 g tert.Butyl 9(S)-amino-octahydro-10-oxo-6H-pyridazo[1,2-a][1,2]diazepin -1-(S)-carboxylat, 3.16 g 2(R,S)-Brom-4-(4-nitrophenyl)butansäure-äthylester und 1.01 g Triäthylamin in 60 ml Acetonitril wird 15 Stunden auf 80°C erhitzt. Das Lösungsmittel wird dann abgedampft, und der Rückstand zwischen Wasser und Methylenchlorid verteilt. Die organische Lösung wird eingedampft, und das zurückbleibende Oel an Silicagel chromatographiert. Eluieren mit Toluol/Aethylacetat (3:2) liefert 1.78 g tert.Butyl 9(S)-[1(R)-äthoxycarbonyl-3 -(4-nitrophenyl)propylamino]-octahydro-10-oxo -6H-pyridazo[1,2-a]-[1,2]diazepin-1(S)-carboxylat als schwach gelbes Oel.

Analyse für $C_{26}H_{38}N_4O_7$:
Berechnet: C: 60.2; H: 7.4; N: 10.8%
Gefunden: C: 60.1; H: 7.4; H: 10.7%.

Danach werden unter Verwendung des gleichen Lösungsmittelsystems 1.93 g tert.Butyl 9(S)-[1(S)-äthoxycarbonyl-3 -(4-nitrophenyl)propylamino]-octahydro-10-oxo -6H-pyridazo[1,2-a][1,2]diazepin-1(S)-carboxylat als schwach gelbes Oel eluiert.

Analyse für $C_{26}H_{38}N_4O_7$:
Berechnet: C: 60.2; H: 7.4; N: 10.8%
Gefunden: C: 60.2; H: 7.3; N: 10.7%.

(B) Eine Lösung von 4.15 g tert.Butyl 9(S)-[1(S)-äthoxycarbonyl-3 -(4-nitrophenyl)propylamino]-octahydro-10-oxo -6H-pyridazo[1,2-a][1,2]diazepin-1(S)-carboxylat in 200 ml Aethanol wird über 10% Palladium auf Kohle bei Raumtemperatur und Atmosphärendruck 3 Stunden hydriert. Der Katalysator wird abfiltriert, und das Volumen des Filtrates durch Abdampfen auf 80 ml eingeengt. 1.76 g 1-Nitroguanyl-3,5-dimethylpyrazol werden zugegeben, und die Lösung 72 Stunden zum Rückfluss erhitzt. Das Lösungsmittel wird durch Abdampfen entfernt, und der Rückstand zwischen Wasser und Methylenchlorid verteilt. Die organische Lösung wird eingedampft, und das zurückbleibende Oel an zwei Silicagelsäulen chromatographiert, wobei die erste Säule mit Aethylacetat und die zweite Säule mit Diäthyläther, enthaltend 5 Volumenprozente Methanol, eluiert wird. Auf diese Weise werden 1.58 g tert.Butyl 9(S)[1-(S)-äthoxycarbonyl-3-[4 -(2-nitroguanidino)phenyl]propylamino]-octahydro-10-oxo -6H-pyridazo [1,2-a][1,2]-diazepin-1(S)-carboxylat als oranger Schaum erhalten.

Analyse für $C_{27}H_{41}N_7O_7$:
Berechnet: C: 56.33; H: 7.2; N: 17.0%
Gefunden: C: 56.14; H: 7.0; N: 17.0%.

(C) Eine Lösung von 0.95 g tert.Butyl 9(S)-[1(S)-äthoxycarbonyl-3 -[4-(2-nitroguanidino)phenyl]-propylamino]-octahydro-10-oxo -6H-pyridazo[1,2-a][1,2]diazepin-1(S)-carboxylat in 4 ml Essigsäure wird mit 12 ml 45%igem Bromwasserstoff in Essigsäure versetzt und 1 Stunde bei 20°C gerührt. Die Lösung wird dann in 500 ml wasserfreien Aether gegossen, 1 Stunde gerührt und danach filtriert, wobei man 0.89 g 9(S)-[1(S)-Aethoxycarbonyl-3-[4 -(2-nitroguanidino)phenyl]propylamino]-octahydro-10-oxo -6H-pyridazo[1,2-a]-[1,2]diazepin-1(S)-carbonsäure-hydrobromid als amorphen weissen Festkörper erhält.

NMR: $\delta_H$ ($CD_3OD$, 300 MHz): 1.33 (3H, t), 1.43 (1H, m), 1.66-1.98 (4H, m), 2.06-2.35 (4H, m), 2.40 (1H, breit, d), 2.60 (1H, m), 2.73-2.96 (2H, m), 3.02 (1H, breit, d), 3.15 (1H, m), 3.47 (1H, m), 4.07 (1H, t), 4.33 (2H, m), 4.80-5.00 (2H, teilweise vom Wasser-Signal verdeckt), 7.29 (2H, d) und 7.35 (2H, d).

MS: m/e 520 (2% [M + H] ) und 211 (100).

<u>Beispiel 4</u>

Eine Lösung von 0.39 g 9(S)-[1(S)-(n-Decyloxycarbonyl)-3-[4 -(2-nitroguanidino)phenyl]propylamino]-octahydro-10-oxo -6H-pyridazo[1,2-a][1,2]diazepin-1(S)-carbonsäure-hydrobromid in einem Gemisch von 16 ml Essigsäure, 4 ml Wasser und 4 Tropfen Bromwasserstoffsäure wird über 10% Palladium auf Kohle bei Raumtemperatur und Atmosphärendruck 48 Stunden hydriert. Der Katalysator wird abfiltriert, und das Filtrat gefriergetrocknet. Der erhaltene Festkörper wird aus Wasser lyophilisiert, wobei man 0.34 g 9(S)-[1(S)-(n-Decyloxycarbonyl)-3 -(4-guanidinophenyl)propylamino]-octahydro-10-oxo -6H-pyridazo[1,2-a][1,2]diazepin - 1(S)-carbonsäure-dihydrobromid als leicht gelblichen amorphen Festkörper erhält.

NMR: $\delta_H$ ($CD_3OD$, 300 MHz): 0.89 (3H, m), 1.21-1.50 (17H, m), 1.66-1.99 (4H, m), 2.03-2.45 (5H, m), 2.61 (1H, m), 2.74-2.98 (2H, m), 3.04 (1H, breit, d), 3.18 (1H, m), 3.49 (1H, m), 4.13 (1H, t), 4.20-4.40 (2H, m), 4.82-5.00 (2H, teilweise vom Wasser-Signal verdeckt), 7.26 (2H, d) und 7.40 (2H, d).

MS: m/e 587 [13% (M + H) ] und 211 (100).

Das als Ausgangsmaterial eingesetzte 9(S)-[1(S)-(n-Decyloxycarbonyl)-3 -[4-(2-nitroguanidinophenyl]-propylamino]-octahydro-10-oxo -6H-pyridazo[1,2-a][1,2]diazepin-1(S)-carbonsäure-hydrobromid wurde wie folgt hergestellt:

(A) Eine Lösung von 2.07 g tert.Butyl 9(S)-[1(S)-äthoxycarbonyl-3 -(4-nitrophenyl)propylamino]-octahydro-10-oxo -6H-pyridazo[1,2-a][1,2]diazepin-1(S)-carboxylat, hergestellt wie in Beispiel 3(A) beschrieben, in 10 ml Aethanol wird mit 10 ml 1M wässriger Natriumhydroxidlösung versetzt. Die erhaltene Lösung wird während 5 Stunden bei 20°C gerührt und danach mit Wasser verdünnt. Das Aethanol wird abgedampft, und die wässrige Lösung auf pH 4 eingestellt und danach mit Methylenchlorid extrahiert. Die organische Lösung wird zu einem gelben Schaum eingedampft. 0,49 g des Schaums werden zu einer 3 Stunden bei 20°C gerührten Lösung von 0.81 g 1,1'-Carbonyldiimidazol und 1.42 g Methyljodid in 5 ml Methylenchlorid gegeben. Danach werden 0,158 g n-Decanol zugegeben, und die Lösung weitere 24 Stunden bei 20°C gerührt. Das Reaktionsgemisch wird zwischen Methylenchlorid und verdünnter Salzsäure verteilt. Die organische Lösung wird eingedampft, und das zurückbleibende Oel an Silicagel chromatographiert. Eluieren mit Diäthyläther/n-Hexan (2:1) liefert 0.19 g tert.Butyl 9(S)-[1(S)-(n-decyloxycarbonyl)-3-(4 -nitrophenyl)propylamino]-octahydro-10-oxo-6H -pyridazo-[1,2-a][1,2]diazepin-1(S)-carboxylat als gelbes Oel.

NMR: $\delta_H$ ($CDCl_3$, 300MHz): 1.88 (3H, m), 1.17-1.41 (17H, m), 1.48 (9H, s), 1.57-1.82 (4H, m), 1.87-2.12 (4H, m), 2.30 (1H, m), 2.50 (1H, m), 2.82-3.13 (4H, m), 3.35 (1H, t), 3.42 (1H, m), 4.05 -4.22 (3H, m), 4.94 (1H, m), 7.37 (2H, d) und 8.14 (2H, d).

MS: m/e 630 (3%, M ), 501 (37), 211 (56), 179 (83), 143 (87) und 57 (100).

(B) Eine Lösung von 2.017 g tert.Butyl 9(S)-[1(S)-(n-decyloxycarbonyl)-3 -(4-nitrophenyl)propylamino]-octahydro-10-oxo -6H-pyridazo[1,2-a][1,2]diazepin-1(S)-carboxylat in 25 ml Aethanol wird über 10% Palladium auf Kohle bei Raumtemperatur und Atmosphärendruck 18 Stunden hydriert. Der Katalysator wird abfiltriert, und das Filtrat eingedampft, wobei man 1.9 g tert.Butyl 9(S)-[3-(4-aminophenyl)-1(S) -(n-decyloxycarbonyl)propylamino]-octahydro-10-oxo -6H-pyridazo[1,2-a][1,2]diazepin-1(S)-carboxylat als farbloses Oel erhält.

Analyse für $C_{34}H_{56}N_4O_5$:
Berechnet: C: 68.0; H: 9.4; N: 9.3%
Gefunden: C: 68.1; H: 9.7; N: 9.2%.

(C) Eine Lösung von 1.67 g tert.Butyl 9(S)-[3-(4-aminophenyl)-1(S) -(n-decyloxycarbonyl)propylamino]-octahydro-10-oxo -6H-pyridazo[1,2-a][1,2]diazepin-1(S)-carboxylat und 0.611 g 1-Nitroguanyl-3,5-dimethylpyrazol in 25 ml Aethanol wird 72 Stunden zum Rückfluss erhitzt. Das Lösungsmittel wird abgedampft, und der Rückstand unter Verwendung von 3% Methanol in Diäthyläther als Eluierungsmittel an Silicagel chromatographiert. Das Material vom Rf-Wert 0.4 (5% Methanol in Diäthyläther) wird zwischen Diäthyläther/n-Hexan und Wasser verteilt. Die organische Phase wird abgetrennt und eingedampft, wobei man 0.76 g tert.Butyl 9(S)-[1(S)-[n-decyloxycarbonyl)-3 -[4-(2-nitroguanidino)phenyl]propylamino]-octahydro-10-oxo -6H-pyridazo[1,2-a][1,2]diazepin-1(S)-carboxylat als weisslichen Schaum erhält.

Analyse für $C_{35}H_{57}N_7O_7$:
Berechnet: C: 61.1; H: 8.35; N: 14.25%
Gefunden: C: 61.0; H: 8.45; N: 14.10%.

(D) Eine Lösung von 0.70 g tert.Butyl 9(S)-[1(S)-(n-decyloxycarbonyl)-3 -[4-(2-nitroguanidino)phenyl]-propylamino]-octahydro-10-oxo -6H-pyridazo[1,2-a][1,2]diazepin-1(S)-carboxylat in 2.5 ml Essigsäure wird mit 8.0 ml 45%igem Bromwasserstoff in Essigsäure versetzt, und die Lösung 1 Stunde bei 20°C gerührt. Die Lösung wird dann in 200 ml wasserfreien Aether gegossen. 2 Stunden gerührt und abfiltriert, wobei man 0.54 g 9(S)-[1(S)-(n-Decyloxycarbonyl)-3 -[4-(2-nitroguanidino)phenyl]propylamino]-octahydro-10-oxo -6H-pyridazo[1,2-a][1,2]diazepin-1(S)-carbonsäure-hydrobromid als weisslichen amorphen Festkörper erhält.

Analyse für $C_{31}H_{49}N_7O_7$. HBr:
Berechnet: C: 52.20; H: 7.07; N: 13.75; Br; 11.21%
Gefunden: C: 51.85; H: 6.95; N: 13.51; Br; 11.23%.

Beispiel 5

Eine Lösung von 0.34 g 9(S)-[1(S)-[[2-(1-Adamantyl)äthoxy]carbonyl]-3 -[4-(2-nitroguanidino)phenyl]-propylamino]-octahydro-10-oxo -6H-pyridazo[1,2-a][1,2]-1(S)-carbonsäure-hydrobromid in 16 ml Essigsäure und 4 ml Wasser, enthaltend einige Tropfen Bromwasserstoffsäure, wird über 10% Palladium auf Kohle bei Raumtemperatur und Atmos phärendruck 48 Stunden hydriert. Der Katalysator wird abfiltriert, und das Filtrat lyophilisiert, wobei man 0.4 g 9(S)-[1(S)-[[2-(1-Adamantyl)äthoxy]carbonyl]-3 -(4-guanidinophenyl)-propylamino]-octahydro-10-oxo -6H-pyridazo[1,2-a][1,2]diazepin -1(S)-carbonsäure-dihydrobromid als weissen amorphen Festkörper erhält.

NMR: $\delta_H$ ($CD_3OD$, 300 MHz): 1.51 (3H, m), 1.60 (6H, s), 1.64-2.03 (13H, m), 2.06-2.36 (4H, m), 2.43 (1H, m), 2.60 (1H, m), 2.74-2.97 (2H, m), 3.04 (1H, m), 3.15 (1H, m), 3.52 (1H, m), 4.02 (1H, t), 4.36 (2H, m), 4.77-5.07 (2H, teilweise vom Wasser-Signal verdeckt), 7.25 (2H, d) und 7.38 (2H, d).

MS: m/e 609 (20% [M + H] ) und 211 (100).

Das als Ausgangsmaterial eingesetzte 9(S)-[1(S)-[[2-(1-Adamantyl)äthoxy]carbonyl]-3--[4-(2-nitroguanidino)phenyl]propylamino]-octahydro-10-oxo -6H-pyridazo[1,2-a][1,2]diazepin-1(S)-carbonsäure-hydrobromid wurde wie folgt hergestellt:

(A) Eine Lösung von 2.95 g tert.Butyl 9(S)-[1(S)-äthoxycarbonyl-3 -(4-nitrophenyl)propylamino]-octahydro-10-oxo -6H-pyridazo[1,2-a][1,2]diazepin-1(S)-carboxylat in 32 ml Aethanol wird mit 28.5 ml einer 0.5M wässrigen Natriumhydroxidlösung 16 Stunden bei 20°C behandelt. Das Gemisch wird mit Wasser verdünnt, und das Aethanol abgedampft. Die wässrige Lösung wird auf pH 6 eingestellt und mit Methylenchlorid extrahiert. Die organische Lösung wird dann eingedampft, wobei man 2.62 g eines gelben Festkörpers erhält. 2.3 g dieses Festkörpers werden zu einer 3 Stunden bei 20°C gerührten Lösung von 3.80 g 1,1'-Carbonyldiimidazol und 6.66 g Methyljodid in 25 ml trockenem Methylenchlorid gegeben. Nach 1 Stunde werden 0.84 g 1-Adamantanäthanol zugegeben, und die Lösung 16 Stunden bei 20°C gerührt. Das Gemisch wird dann filtriert, und das Filtrat mit Methylenchlorid verdünnt, nacheinander mit 2M wässriger Salzsäure, Wasser, 5%iger wässriger Natriumbicarbonatlösung und Wasser gewaschen und dann

eingedampft. Chromatographie an zwei Silicagelsäulen unter Verwendung von Chloroform/Methanol/Essigsäure/Wasser (120:15:3:2) zum Eluieren der ersten Säule und Diäthyläther zum Eluieren der zweiten Säule liefert 1.84 g tert.Butyl 9(S)-[1(S)-[[2-(1-adamantyl)äthoxy]carbonyl]-3 -(4-nitrophenyl)propylamino]-octahydro-10-oxo-6H -pyridazo[1,2-a][1,2]diazepin-1(S)-carboxylat als schwach gelbes Oel.

(B) Eine Lösung von 1.84 g tert.Butyl 9(S)-[1(S)-[[2-(1-adamantyl)äthoxy]carbonyl]-3 -(4-nitrophenyl)-propylamino]-octahydro-10-oxo-6H -pyridazo[1,2-a][1,2]diazepin-1(S)-carboxylat in 25 ml Aethanol wird über 10% Palladium auf Kohle bei Raumtemperatur und Atmosphärendruck 16 Stunden hydriert. Der Katalysator wird abfiltriert, und das Filtrat eingedampft, wobei man 1,8 g eines schwach gelben Oels erhält. Eine Lösung von 1,6 g dieses Oels und 0.56 g 1-Nitroguanyl-3,5-dimethylpyrazol in 20 ml Aethanol wird 72 Stunden unter Rühren zum Rückfluss erhitzt. Das Gemisch wird danach eingedampft, und der Rückstand an zwei Silicagelsäulen chromatographiert, wobei zum Eluieren der ersten Säule Diäthyläther, enthaltend 3% Volumenprozente Methanol, und zum Eluieren der zweiten Säule Aethylacetat verwendet werden. Auf diese Weise erhält man 0.50 g tert.Butyl 9(S)-[1(S)-[[2-(1-adamantyl)äthoxy]carbonyl]-3-[4 -(2-nitroguanidino)phenyl]propylamino]-octahydro-10-oxo -6H-pyridazo[1,2-a][1,2]diazepin-1(S)-carboxylat als weissen Schaum.

Analyse für $C_{37}H_{55}N_7O_7$:
Berechnet: C: 62.60; H: 7.81; N: 13.81%
Gefunden: C: 62.52; H: 7.82; N: 13.86%.

(C) Eine Lösung von 0.45 g tert.Butyl 9(S)-[1(S)-[[2-(1-adamantyl)äthoxy]carbonyl]-3 -[4-(2-nitroguanidino)phenyl]propylamino]-octahydro-10-oxo -6H-pyridazo[1,2-a][1,2]diazepin-1(S)-carboxylat in 1.5 ml Essigsäure wird mit 5 ml 45%igem Bromwasserstoff in Essigsäure versetzt, und das Gemisch 1 Stunde bei 20°C gerührt. Die Reaktionslösung wird in 200 ml wasserfreien Diäthyläther gegossen, 1 Stunde gerührt und danach filtriert, wobei man 0.53 g 9(S)-[1(S)-[[2-(1-Adamantyl)äthoxy]carbonyl]-3 -[4-(2-nitroguanidino)-phenyl]propylamino]-octahydro-10-oxo -6H-pyridazo[1,2-a][1,2]diazepin-1(S)-carbonsäure-hydrobromid als weissen Festkörper erhält.

NMR: $\delta_H$ ($CD_3OD$, 300MHz): 1.50 (3H, m), 1.58 (6H, s), 1.63-2.00 (13H, m), 2.07-2.36 (4H, m), 2.41 (1H, m), 2.61 (1H, m), 2.74-2.98 (2H, m), 3.05 (1H, breit, d), 3.17 (1H, m), 3.48 (1H, m), 4.06 (1H, t), 4.33 (2H, m), 4.82-4.97 (2H, teilweise vom Wasser-Signal verdeckt) und 7.32 (4H, m).
MS: m/e 654 (3% [M + H] ) und 211 (100).

Beispiel 6

Eine Lösung von 0.48 g 9(S)-[N-Benzyl-1(S)-äthoxycarbonyl)-3 -[4-(2-nitroguanidino)phenyl]-propylamino]-octahydro-10-oxo -6H-pyridazo[1,2-a][1,2]diazepin -1(S)-carbonsäure-pivaloyloxymethylester in 20 ml Essigsäure und 5 ml Wasser wird über 10% Palladium auf Kohle bei Raumtemperatur und Atmosphärendruck 30 Stunden hydriert. Der Katalysator wird abfiltriert, und das Filtrat lyophilisiert. Der dabei erhaltene Gummi wird an Silicagel unter Verwendung von Chloroform/Methanol/Essigsäure/Wasser (120:15:3:2) als Eluierungsmittel chromatographiert, wobei man 0.19 g 9(S)-[1(S)-Aethoxycarbonyl-3-(4-guanidinophenyl)propylamino] -octahydro-10-oxo-6H-pyridazo[1,2-a][1,2]diazepin -1(S)-carbonsäure-pivaloyloxymethylester-acetat als weisslichen amorphen Festkörper erhält.

Analyse für $C_{29}H_{44}N_6O_7$. 1:1 $C_2H_4O_2$:
Berechnet: C: 57.39; H: 7.46; N: 12.95%
Gefunden: C: 56.90; H: 7.52; N: 12.87%.

NMR: $\delta_H$ ($CDCl_3$, 300 MHz): 1.21 (9H, s), 1.28 (3H, t), 1.34-2.12 (9H, m), 1.97 (3H, s), 2.28 (1H, m), 2.32 (1H, m), 7.74 (2H, m), 2.95 (1H, m), 3.08 (1H, m), 3.31 (1H, t), 3.35 (1H, m), 4.14 (1H, m), 4.20 (2H, m), 5.02 (1H, m), 5.74 (1H, d), 5.83 (1H, d), 7.14 (2H, d) und 7.24 (2H, d).
MS: m/e 589 (30%, [M + H] ), 211 (80) und 149 (100).

Der als Ausgangsmaterial eingesetzte 9(S)-[N-Benzyl-1(S)-äthoxycarbonyl-3 -[4-(2-nitroguanidino)-phenyl)propylamino]-octahydro-10-oxo -6H-pyridazo[1,2-a][1,2]diazepin -1(S)-carbonsäure-pivaloyloxymethylester wurde wie folgt hergestellt:

(A) 5.09 g tert.Butyl 9(S)-amino-octahydro-10-oxo -6H-pyridazo[1,2-a][1,2]diazepin-1(S)-carboxylat, 1.91 g Benzaldehyd und 3 g 3A Molekularsieb in 54 ml Aethanol werden 3 Stunden gerührt und danach über 5% Palladium auf Kohle bei Raumtemperatur und Atmosphärendruck 1,75 Stunden hydriert. Der Katalysator wird abfiltriert, und das Filtrat eingedampft. Chromatographie des Rückstands an Silicagel unter Verwendung von Diäthyläther/Methanol (39:1) als Eluierungsmittel liefert 5.25 g tert.Butyl 9(S)-benzylamino-octahydro-10-oxo -6H-pyridazo[1,2-a][1,2]diazepin-1(S)-carboxylat als schwach gelbes Oel.

NMR: $\delta_H$ ($CDCl_3$, 300 MHz): 1.37 (1H, m), 1.47 (10H, m), 1.65-1.84 (3H, m), 1.92 (1H, m), 2.07 (1H, m), 2.31 (1H, breit, d), 2.52 (1H, m), 2.94 (1H, breit, d), 3.07 (1H, m), 3.28 (1H, breit, s), 3.41 (1H, m), 3.68 (1H, d), 3.89 (1H, d), 4.23 (1H, t), 4.97 (1H, m) und 7.20-7.44 (5H, m).

(B) Eine Lösung von 1.87 g tert.Butyl 9(S)-benzylamino-octahydro-10-oxo -6H-pyridazo[1,2-a][1,2]-diazepin-1(S)-carboxylat, 1.93 g 2(R)-Trifluormethansulfonyloxy -4-(4-nitrophenyl)butansäure-äthylester und 0.7 g Triäthylamin in 5 ml Acetonitril wird 2 Stunden bei 20°C gerührt. Weitere 0.2 g 2(R)-Trifluormethansulfonyloxy -4-(4-nitrophenyl)butansäure-äthylester und 0.05 g Triäthylamin werden zugegeben, und die Lösung danach 16 Stunden bei 20°C stehengelassen. Die Lösungsmittel werden danach abgedampft, und der Rückstand zwischen Wasser und Methylenchlorid verteilt. Eindampfen der organischen Lösung und Chromatographie an Silicagel unter Verwendung von Diäthyläther/n-Hexan (1:1) als Eluierungsmittel liefert 2.44 g tert.Butyl 9(S)-[N-benzyl-1(S)-äthoxycarbonyl-3 -(4-nitrophenyl)propylamino]-octahydro-10-oxo -6H-pyridazo[1,2-a][1,2]diazepin-1(S)-carboxylat als gelbliches Oel.

Analyse für $C_{33}H_{44}N_4O_7$:
Berechnet: C: 65.11; H: 7.29; N: 9.20%
Gefunden: C: 65.41; H: 7.15; N: 9.20%.

(C) Eine Lösung von 2.0 g tert.Butyl 9(S)-[N-benzyl-1(S)-äthoxycarbonyl-3 -(4-nitrophenyl)propylamino]-octahydro-10-oxo -6H-pyridazo[1,2-a][1,2]diazepin-1(S)-carboxylat in 10 ml Trifluoressigsäure wird bei 20°C 2,5 Stunden gerührt und danach 16 Stunden bei 0°C stehengelassen. 20 ml Toluol werden zugegeben, und die Lösung eingedampft. Der Rückstand wird in Methylenchlorid gelöst, mit Wasser und Natriumchloridlösung gewaschen und dann eingedampft. Chromatographie an Silicagel unter Verwendung von Methylenchlorid/Methanol (19:1) als Eluierungsmittel liefert 0.9 g 9(S)-[N-Benzyl-1(S)-äthoxycarbonyl-3 -(4-nitrophenyl)propylamino]-octahydro-10-oxo -6H-pyridazo[1,2-a][1,2]diazepin-1(S)-carbonsäure als gelbliches Oel.

NMR: $\delta_H$ ($CDCl_3$, 300 MHz): 1.33 (4H, m), 1.62 (1H, m), 1.71-2.09 (7H, m), 2.40 (1H, m), 2.45-2.59 (2H, m), 2.66 (1H, m), 3.00 (1H, breit, d), 3.17 (1H, m), 3.29 (2H, m), 4.01-4.20 (2H, m), 4.23 (1H, d), 4.39 (1H, d), 4.75 (1H, t), 4.84 (1H, m), 7.13 (2H, d), 7.19-7.40 (5H, m) und 8.04 (2H, d).
MS: m/e 553 (2%, [M + H] ) und 211 (100).

(D) Eine Lösung von 2.68 g 9(S)-[N-Benzyl-1(S)-äthoxycarbonyl-3 -(4-nitrophenyl)propylamino]-octahydro-10-oxo -6H-pyridazo[1,2-a][1,2]diazepin-1(S)-carbonsäure in 10 ml Aceton wird mit 0.32 g Kaliumhydroxid in 0.5 ml Wasser, 0.73 g Chlormethylpivalat und 0.12 g Natriumjodid versetzt. Das Reaktionsgemisch wird 5 Stunden zum Rückfluss erhitzt und danach zwischen Wasser und Methylenchlorid verteilt. Die organische Phase wird eingedampft, und der Rückstand an Silicagel unter Verwendung von Diäthyläther/n-Hexan (1:1) als Eluierungsmittel chromatographiert, wobei man 1.72 g 9(S)-[N-Benzyl-1(S)-äthoxycarbonyl-3 -(4-nitrophenyl)propylamino]-octahydro-10-oxo -6H-pyridazo[1,2-a][1,2]diazepin -1(S)-carbonsäure-pivaloyloxymethylester als gelbliches Oel erhält.

Analyse für $C_{35}H_{46}N_4O_9$:
Berechnet: C: 63.05; H: 6.95; N: 8.40%
Gefunden: C: 62.81; H: 6.84; N: 8.22%.

(E) Ein Gemisch von 1.66 g 9(S)-[N-Benzyl-1(S)-äthoxycarbonyl-3 -(4-nitrophenyl)propylamino]-octahydro-10-oxo -6H-pyridazo[1,2-a][1,2]diazepin -1(S)-carbonsäure-pivaloyloxymethylester und 0.97 g Zinkpuder in 35 ml 85%iger Essigsäure wird 3 Stunden bei 20°C gerührt. Das überschüssige Zink wird abfiltriert, und das Filtrat eingedampft. Der Rückstand wird zwischen Wasser und Methylenchlorid verteilt, und die organische Phase erneut eingedampft. Das zurückbleibende Oel wird in 25 ml Aethanol aufgenommen, und die Lösung mit 0.55 g 1-Nitroguanyl-3,5-dimethylpyrazol ver setzt. Die Lösung wird dann unter Rühren 72 Stunden zum Rückfluss erhitzt. Weitere 0.55 g 1-Nitroguanyl-3,5-dimethylpyrazol werden zugegeben, und das Gemisch weitere 24 Stunden unter Rühren zum Rückfluss erhitzt. Das Gemisch wird danach zur Trockene eingedampft, und der Rückstand an Silicagel unter Verwendung von Diäthyläther/Methanol (39:1) als Eluierungsmittel chromatographiert, wobei man 0.58 g 9(S)-[N-Benzyl-1(S)-äthoxycarbonyl-3-[4 -(2-nitroguanidino)phenyl]propylamino]-octahydro-10-oxo -6H-pyridazo[1,2-a][1,2]-diazepin -1(S)-carbonsäure-pivaloyloxymethylester als weisslichen Schaum erhält.

Analyse für $C_{36}H_{49}N_7O_9$:
Berechnet: C: 59.74; H: 6.82; N: 13.55%;
Gefunden: C: 59.75; H: 6.85; N: 13.38%.

Die folgenden Beispiele veranschaulichen pharmazeutische Präparate, enthaltend 9(S)-[1(S)-Carboxy-3 -(4-guanidinophenyl)propylamino]-octahydro-10-oxo -6H-pyridazo[1,2-a][1,2]diazepin -1(S)-carbonsäure-dihydrobromid (Verbindung A) als aktiven Wirkstoff.

Beispiel A

Tabletten, enthaltend die folgenden Bestandteile, können in herkömmlicher Weise hergestellt werden:

| Bestandteile | | Pro Tablette |
|---|---|---|
| Verbindung A | | 10.0 mg |
| Lactose | | 125.0 mg |
| Maistärke | | 75.0 mg |
| Talk | | 4.0 mg |
| Magnesiumstearat | | 1.0 mg |
| | Tablettengewicht | 215.0 mg |

Beispiel B

Kapseln, enthaltend die folgenden Bestandteile, können in herkömmlicher Weise hergestellt werden:

| Bestandteile | | Pro Kapsel |
|---|---|---|
| Verbindung A | | 25.0 mg |
| Lactose | | 150.0 mg |
| Maisstärke | | 20.0 mg |
| Talk | | 5.0 mg |
| | Kapselfüllgewicht | 200.0 mg |

**Ansprüche**

1. Pyridazodiazepinderivate der allgemeinen Formel

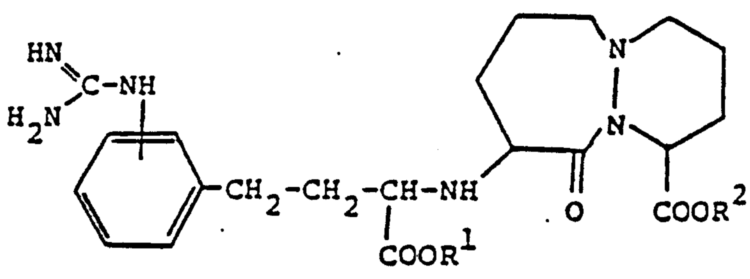

I

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl) bedeuten,
und pharmazeutisch verwendbare Salze davon.

2. Pyridazodiazepinderivate gemäss Anspruch 1, worin $R^1$ Wasserstoff, $C_1$-$C_{10}$-Alkyl oder Adamantyl-($C_1$-$C_4$-alkyl) bedeutet.

3. Pyridazodiazepinderivate gemäss Anspruch 2, worin $R^1$ Wasserstoff, Aethyl, n-Decyl oder 1-Adamantyläthyl bedeutet.

4. Pyridodiazepinderivate gemäss einem der Ansprüche 1-3, worin $R^2$ Wasserstoff oder ($C_2$-$C_6$-Alkanoyloxy)-($C_1$-$C_4$-alkyl) bedeutet.

5. Pyridazodiazepinderivate gemäss Anspruch 4, worin $R^2$ Wasserstof oder Pivaloyloxymethyl bedeutet.

6. Pyridazodiazepinderivate gemäss einem der Ansprüche 1-5, worin die Guanidinogruppe $H_2N$-C(NH)-NH-sich in para-Stellung des Phenylrings befindet.

7. Pyridazodiazepinderivate gemäss einem der Ansprüche 1-6, worin $R^1$ Wasserstoff, Aethyl, n-Decyl oder 1-Adamantyläthyl und $R^2$ Wasserstoff oder Pivaloyloxymethyl bedeuten und die Guanidinogruppe $H_2N$-C(NH)-NH-sich in para-Stellung des Phenylrings befindet.

8. 9(S)-[1(S)-Carboxy-3-(4-guanidinophenyl)propylamino]-octahydro-10-oxo-6H-pyridazo[1,2-a][1,2]-diazepin-1(S)-carbonsäure und pharmazeutisch verwendbare Salze davon.

9. 9(S)-[1(S)-Carboxy-3-(4-guanidinophenyl)propylamino]-octahydro-10-oxo-6H-pyridazo[1,2-a][1,2]-diazepin-1(S)-carbonsäure-dihydrobromid.

10. 9(S)-[1(S)-Aethoxycarbonyl-3-(4-guanidinophenyl)-propylamino]-octahydro-10-oxo-6H-pyridazo[1,2-a][1,2]diazepin-1(S)-carbonsäure,
9(S)-[1(S)-(n-Decyloxycarbonyl)-3-(4-guanidinophenyl)propylamino]-octahydro-10-oxo-6H-pyridazo[1,2-a]-[1,2]diazepin-1(S)-carbonsäure,
9(S)-[1(S)-[[2-(1-Adamantyl)äthoxy]carbonyl]-3-(4-guanidinophenyl)propylamino]-octahydro-10-oxo-6H-pyridazo[1,2-a][1,2]diazepin-1(S)-carbonsäure oder
9(S)-[1(S)-Aethoxycarbonyl-3-(4-guanidinophenyl)propylamino]-octahydro-10-oxo-6H-pyridazo[1,2-a][1,2]-diazepin-1(S)-carbonsäure-pivaloyloxymethylester
oder ein pharmazeutisch verwendbares Salz davon.

11. Verbindungen der allgemeinen Formel

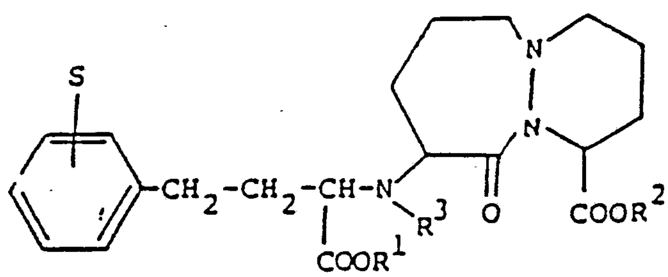

IX

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl), $R^3$ Wasserstoff oder Benzyl und S Nitro, Amino oder 2-Nitroguanidino bedeuten.

12. Pyridazodiazepinderivat gemäss einem der Ansprüche 1-10 zur Anwendung als therapeutischer Wirkstoff.

13. Pyridazodiazepinderivat gemäss einem der Ansprüche 1-10 zur Anwendung als Antihypertensivum.

14. Verfahren zur Herstellung eines Pyridazodiazepinderivates gemäss einem der Ansprüche 1-10, dadurch gekennzeichnet, dass man

a) eine Verbindung der allgemeinen Formel

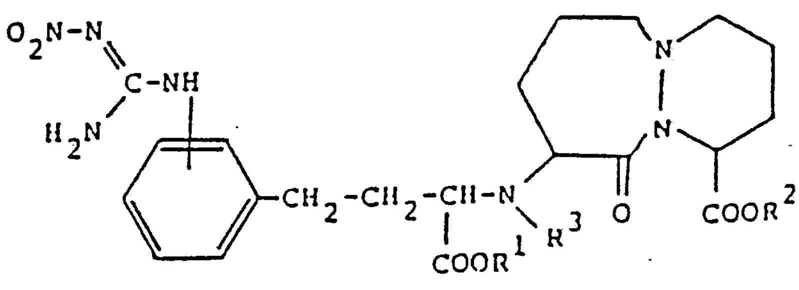

II

worin $R^1$ und $R^2$ die oben angegebene Bedeutung besitzen und $R^3$ Wasserstoff oder Benzyl bedeutet, in einem sauren Medium katalytisch hydriert und ein erhaltenes nicht-pharmazeutisch verwendbares Salz einer Verbindung der Formel I in eine Verbindung der Formel I überführt, oder

b) zur Herstellung einer Verbindung der Formel I, worin $R^1$ und/oder $R^2$ Wasserstoff bedeuten, eine Verbindung der Formel I, worin $R^1$ und/oder $R^2$ $C_1$-$C_{10}$-Alkyl bedeuten, mit einer Säure und/oder einer Base behandelt, und/oder

c) erwünschtenfalls, ein erhaltenes Gemisch von diastereomeren Racematen in die entsprechenden diastereomeren Racemate oder optisch reinen Diastereomeren auftrennt, und/oder

d) erwünschtenfalls, ein erhaltenes Racemat in die beiden optischen Antipoden auftrennt, und

e) erwünschtenfalls, eine erhaltene Verbindung der Formel I in ein pharmazeutisch verwendbares Salz oder ein pharmazeutisch verwendbares Säureadditionssalz einer Verbindung der Formel I in eine Verbindung der Formel I überführt.

15. Arzneimittel, enthaltend ein Pyridazodiazepinderivat gemäss einem der Ansprüche 1-10 und ein therapeutisch inertes Excipiens.

16. Antihypertensivum, enthaltend ein Pyridazodiazepinderivat gemäss einem der Ansprüche 1-10 und ein therapeutisch inertes Excipiens.

17. Verwendung eines Pyridazodiazepinderivates gemäss einem der Ansprüche 1-10 zur Herstellung eines Antihypertensivums.

Patentansprüche für den folgenden Vertragsstaat: ES

1. Verfahren zur Herstellung eines Pyridazodiazepinderivates der allgemeinen Formel

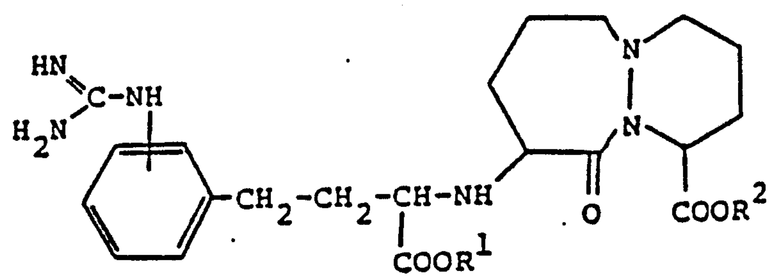

I

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl) bedeuten,
und pharmazeutisch verwendbaren Salzen davon, dadurch gekennzeichnet, dass man

a) eine Verbindung der allgemeinen Formel

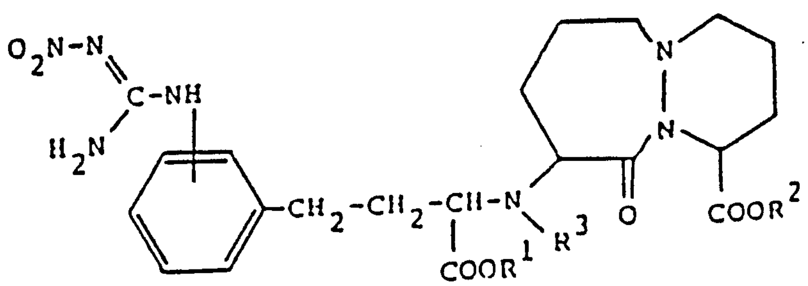

II

worin $R^1$ und $R^2$ die oben angegebene Bedeutung besitzen und $R^3$ Wasserstoff oder Benzyl bedeutet, in einem sauren Medium katalytisch hydriert und ein erhaltenes nicht-pharmazeutisch verwendbares Salz einer Verbindung der Formel I in eine Verbindung der Formel I überführt, oder

b) zur Herstellung einer Verbindung der Formel I, worin $R^1$ und/oder $R^2$ Wasserstoff bedeuten, eine Verbindung der Formel I, worin $R^1$ und/oder $R^2$ $C_1$-$C_{10}$-Alkyl bedeuten, mit einer Säure und/oder einer Base behandelt, und/oder

c) erwünschtenfalls, ein erhaltenes Gemisch von diastereomeren Racematen in die entsprechenden diastereomeren Racemate oder optisch reinen Diastereomeren auftrennt, und/oder

d) erwünschtenfalls, ein erhaltenes Racemat in die beiden optischen Antipoden auftrennt, und

e) erwünschtenfalls, eine erhaltene Verbindung der Formel I in ein pharmazeutisch verwendbares Salz oder ein pharmazeutisch verwendbares Säureadditionssalz einer Verbindung der Formel I in eine Verbindung der Formel I überführt.

2. Verfahren gemäss Anspruch 1, worin $R^1$ Wasserstoff, $C_1$-$C_{10}$-Alkyl oder Adamantyl-($C_1$-$C_4$-alkyl) bedeutet.

3. Verfahren gemäss Anspruch 2, worin $R^1$ Wasserstoff, Aethyl, n-Decyl oder 1-Adamantyläthyl bedeutet.

4. Verfahren gemäss einem der Ansprüche 1-3, worin $R^2$ Wasserstoff oder ($C_2$-$C_6$-Alkanoyloxy)-($C_1$-$C_4$-alkyl) bedeutet.

5. Verfahren gemäss Anspruch 4, worin $R^2$ Wasserstoff oder Pivaloyloxymethyl bedeutet.

6. Verfahren gemäss einem der Ansprüche 1-4, worin die Guanidinogruppe $H_2N$-C(NH)-NH-sich in para-Stellung des Phenylrings befindet.

7. Verfahren gemäss einem der Ansprüche 1-6, worin $R^1$ Wasserstoff, Aethyl, n-Decyl oder 1-Adamantyläthyl und $R^2$ Wasserstoff oder Pivaloyloxymethyl bedeuten und die Guanidinogruppe $H_2N$-C(NH)-NH-sich in para-Stellung des Phenylrings befindet.

8. Verfahren gemäss Anspruch 1, dadurch gekennzeichnet, dass man 9(S)-[1(S)-Carboxy-3-(4-guanidinophenyl)propylamino] -octahydro-10-oxo-6H-pyridazo[1,2-a][1,2]diazepin -1(S)-carbonsäure oder ein pharmazeutisch verwendbares Salz davon herstellt.

9. Verfahren gemäss Anspruch 1, dadurch gekennzeichnet, dass man 9(S)-[1(S)-Carboxy-3-(4-guanidinophenyl)propylamino] -octahydro-10-oxo-6H-pyridazo[1,2-a][1,2]diazepin -1(S)-carbonsäure-dihydrobromid herstellt.

10. Verwendung eines Pyridazodiazepinderivates der Formel I in Anspruch 1 oder eines pharmazeutisch verwendbaren Salzes davon zur Herstellung eines Antihypertensivums.

Patentansprüche für den folgenden Vertragsstaat: GR

1. Verfahren zur Herstellung eines Pyridazodiazepinderivates der allgemeinen Formel

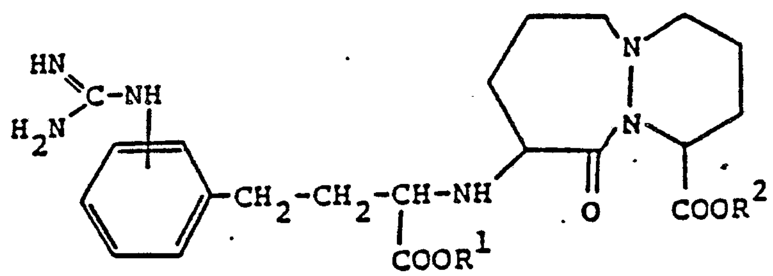

I

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl) bedeuten,
und pharmazeutisch verwendbaren Salzen davon, dadurch gekennzeichnet, dass man

a) eine Verbindung der allgemeinen Formel

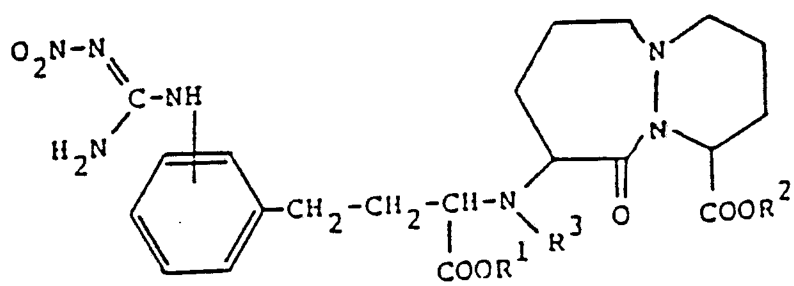

II

worin $R^1$ und $R^2$ die oben angegebene Bedeutung besitzen und $R^3$ Wasserstoff oder Benzyl bedeutet, in einem sauren Medium katalytisch hydriert und ein erhaltenes nicht-pharmazeutisch verwendbares Salz einer Verbindung der Formel I in eine Verbindung der Formel I überführt, oder

b) zur Herstellung einer Verbindung der Formel I, worin $R^1$ und/oder $R^2$ Wasserstoff bedeuten, eine Verbindung der Formel I, worin $R^1$ und/oder $R^2$ $C_1$-$C_{10}$-Alkyl bedeuten, mit einer Säure und/oder einer Base behandelt, und/oder

c) erwünschtenfalls, ein erhaltenes Gemisch von diastereomeren Racematen in die entsprechenden diastereomeren Racemate oder optisch reinen Diastereomeren auftrennt, und/oder

d) erwünschtenfalls, ein erhaltenes Racemat in die beiden optischen Antipoden auftrennt, und

e) erwünschtenfalls, eine erhaltene Verbindung der Formel I in ein pharmazeutisch verwendbares Salz oder ein pharmazeutisch verwendbares Säureadditionssalz einer Verbindung der Formel I in eine Verbindung der Formel I überführt.

2. Verfahren gemäss Anspruch 1, worin $R^1$ Wasserstoff, $C_1$-$C_{10}$-Alkyl oder Adamantyl-($C_1$-$C_4$-alkyl) bedeutet.

3. Verfahren gemäss Anspruch 2, worin $R^1$ Wasserstoff, Aethyl, n-Decyl oder 1-Adamantyläthyl bedeutet.

4. Verfahren gemäss einem der Ansprüche 1-3, worin $R^2$ Wasserstoff oder ($C_2$-$C_6$-Alkanoyloxy)-($C_1$-$C_4$-alkyl) bedeutet.

5. Verfahren gemäss Anspruch 4, worin $R^2$ Wasserstoff oder Pivaloyloxymethyl bedeutet.

6. Verfahren gemäss einem der Ansprüche 1-5, worin die Guanidinogruppe $H_2N$-C(NH)-NH-sich in para-Stellung des Phenylrings befindet.

7. Verfahren gemäss einem der Ansprüche 1-6, worin $R^1$ Wasserstoff, Aethyl, n-Decyl oder 1-Adamantyläthyl und $R^2$ Wasserstoff oder Pivaloyloxymethyl bedeuten und die Guanidinogruppe $H_2N$-C(NH)-NH-sich in para-Stellung des Phenylrings befindet.

8. Verfahren gemäss Anspruch 1, dadurch gekennzeichnet, dass man 9(S)-[1(S)-Carboxy-3-(4-guanidinophenyl)propylamino] -octahydro-10-oxo-6H-pyridazo[1,2-a][1,2]diazepin -1(S)-carbonsäure oder ein pharmazeutisch verwendbares Salz davon herstellt.

9. Verfahren gemäss Anspruch 1, dadurch gekennzeichnet, dass man 9(S)-[1(S)-Carboxy-3-(4-guanidinophenyl)propylamino] -octahydro-10-oxo-6H-pyridazo[1,2-a][1,2]diazepin -1(S)-carbonsäure-dihydrobromid herstellt.

10. Verwendung eines Pyridazodiazepinderivates der Formel I in Anspruch 1 oder eines pharmazeutisch verwendbaren Salzes davon zur Herstellung eines Antihypertensivums.

11. Verbindungen der allgemeinen Formel

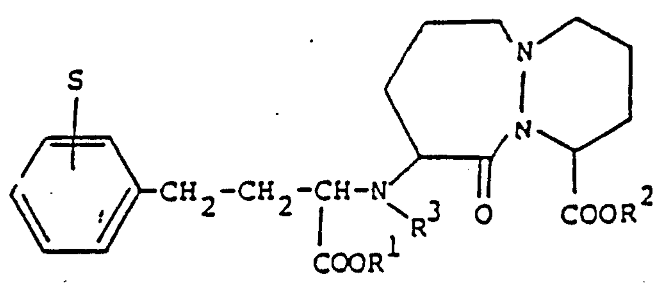

IX

worin $R^1$ und $R^2$ je Wasserstoff, $C_1$-$C_{10}$-Alkyl, Adamantyl-($C_1$-$C_4$-alkyl) oder ($C_2$-$C_6$-Alkanoyloxy)-($C_1$-$C_4$-alkyl), $R^3$ Wasserstoff oder Benzyl und S Nitro, Amino oder 2-Nitroguanidino bedeuten.